# EUROPEAN PATENT APPLICATION

(11) **EP 1 190 844 A1**
(43) Date of publication of application: **27.03.2002**
(21) Application number: 01921773.6
(22) Date of filing: 28.03.2001
(51) Int. Cl.: B32B 5/24, A61F 13/537

(54) **WATER-ABSORBING COMPOSITE SHEET**

(30) Priority: 14.04.2000 JP 2000113056
(71) Applicant: TEIJIN LIMITED, Osaka-shi Osaka 541-0054 (JP)
(72) Inventor: TAKATERA, Yoshikazu, Teijin Ltd., Osaka Res. Ctr., Ibaraki-shi, Osaka 567-0006 (JP); HORIKAWA, Naoki, Teijin Ltd., Osaka Res. Ctr., Ibaraki-shi, Osaka 567-0006 (JP)
(74) Representative: Hallybone, Huw George
(86) International application number: JP0102576
(87) International publication number: WO0178976

(57) **Abstract**

A comfortable water absorbent composite sheet which is a water absorbent sheet comprising a water absorbent layer, a water holding layer and a water impermeable layer successively arranged from the skin side, wherein the composite water absorbent sheet has a sufficient amount of water absorption with slight water-rewet to the skin by arranging a water-backflow preventing layer comprising a water impermeable sheet material having water penetrating holes between the water absorbent layer and the water holding layer and providing the total area ratio of the water penetrating holes accounting for 3% or above and below 40% of the whole area of the water-backflow preventing layer.

## Description

### Technical Field

The present invention relates to a composite sheet having a four-layer structure comprising a water absorbent layer, a water-backflow preventing layer, a water holding layer and a water impermeable layer and relates to a composite water absorbent sheet suitable as diapers for infants or nursing care, urine pads and bed pads or urine absorbent sheets for pets.

### Background Art

Sheets for absorbing excrements containing body fluids discharged from human bodies have hitherto been mostly the disposable type using a macromolecular water absorbent polymer material due to its convenience for use. The conventional products have been generally spread as paper diapers due to slight water-rewet and excellence in comfortableness and further lightweight and good handleability in spite of a large amount of water absorption. The paper diapers, however, have many problems in aspects of environments, since there are problems about an increase in amount of refuse because of disposing the diapers after use thereof only once, and, because paper diapers absorbing water are hard to burn, fears of possibly remaining unburned in some incineration furnaces at low combustion temperatures and of promoting the production of noxious substances such as dioxin from chlorine-containing wastes in furnaces due to reduction of the combustion temperatures of the furnaces.

On the other hand, the so-called reusable cloth diapers are generally woven fabrics using cotton materials, and, however, there are problems that the cloth diapers are cool and uncomfortable due to great water-rewet, in spite of good water absorbability.

### Disclosure of the Invention

It is an object of the present invention to provide a washable and reutilizable comfortable composite water absorbent sheet capable of eliminating the above problems possessed by the prior art and having slight water-rewet and a slight cool touch even in contact with skin after absorbing water.

As a result of intensive studies made to achieve the above object, the present inventors have cleared up that a desired water absorbent composite sheet is obtained by arranging a water-backflow preventing layer between a water absorbent layer on the surface and a water holding layer. Thereby, the present invention has been completed.

Namely, according to the present invention, there is provided a water absorbent composite sheet which is a water absorbent sheet comprising (a) a water absorbent layer, (b) a water holding layer and (c) a water impermeable layer successively arranged from the skin side and characterized in that (d) a water-backflow preventing layer comprising a water impermeable sheet material having water penetrating holes is arranged between the layer (a) and the layer (b) and the total area ratio of the water penetrating holes accounts for 3% or above and below 40% of the whole area of the layer (d).

At least one layer of the above layers (a) and (b) is preferably bound through the layer (d). The binding part of the layers (a) and (b) is more preferably bound through the water penetrating holes of the layer (d). The binding method is preferably one for binding using a sewing thread.

### Brief Description of Drawings

Fig. 1 is a schematic drawing illustrating the structure of cross section in the central part of the composite water absorbent sheet of the present invention.
Fig. 2 is a schematic drawing illustrating the cross-sectional view in the central part of the composite water absorbent sheet wherein the water absorbent layer is bound through water penetrating holes of a water-backflow preventing layer to a water holding layer, and Fig. 3 is a schematic drawing illustrating a cross-sectional structure thereof.
Fig. 4 is an exploded view illustrating one example of the shape of the water absorbent layer, water-backflow preventing layer, water holding layer and water impermeable layer constituting the water absorbent composite sheet of the present invention.
Fig. 5 is a plane view illustrating one example of a water impermeable sheet material constituting the composite water absorbent sheet of the present invention.
Fig. 6 is a perspective view illustrating one example of a urine pad using the water absorbent composite sheet of the present invention.

### Best Mode for Carrying Out the Invention

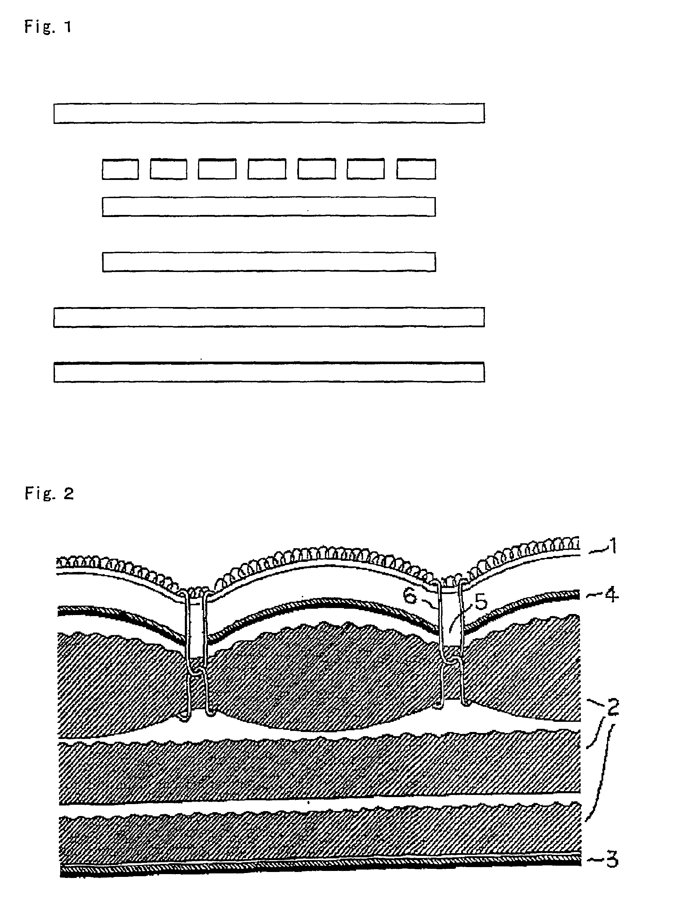

As illustrated in, for example, Fig. 1, the composite water absorbent sheet of the present invention has a structure wherein (a) a water absorbent layer 1, (d) a water-backflow preventing layer 4, (b) a water holding layer 2 and (c) a water impermeable layer 3 are successively arranged from the direction in which water is dropped.

In the composite water absorbent sheet of the present invention, since it is necessary for the water absorbent layer (a) to absorb water once but to transfer water, without subsequently holding water, through water penetrating holes of the next water-backflow preventing layer (d) to the water holding layer (b) and to maintain the surface of the water absorbent layer (a) after absorbing water in dry touch as much as possible, the surface preferably has unevennesses depending on a fabric structure. Specifically, the surface preferably has the unevennesses depending on a raising treatment, cut piles, loop piles, ridges and a mesh structure.

Furthermore, natural fibers, synthetic fibers and a combination thereof can be used as a material constituting the water absorbent layer (a). The surface dry touch is better with increasing mixture ratio of the synthetic fibers. On the other hand, the total fineness of yarns constituting the surface of the water absorbent layer (a) is preferably 222.2 dtex or below, and the single filament fineness is preferably 5.5 dtex or below in order to improve the skin touch and not to cause skin irritation.

A fabric structure having sufficient water absorbent sites among fibers due to the capillarity is required for the structure of the water holding layer (b) in order to absorb and hold enough water, and a knitted structure is preferable.

The water-holding capacity in the water holding layer (b) is preferably 100% or above based on the own weight of the water holding layer, more preferably 180% or above based on the own weight. The water holding layer (b) is not always one layer and may be composed of plural layers according to the required water-holding capacity. Optional synthetic fibers, natural fibers and a combination thereof can be used as a material of yarns used in the water holding layer (b). The water-holding capacity and water absorbability of the water holding layer (b) are preferably unlowered by repetition of washing. For example, the use of a hydrophilic processing agent as a finishing agent after washing is cited in the case where synthetic fibers are used.

The water-backflow preventing layer (d) is arranged between the water absorbent layer (a) 1 and the water holding layer (b) 2 as illustrated in Figures 1, 2, 3 and 4.

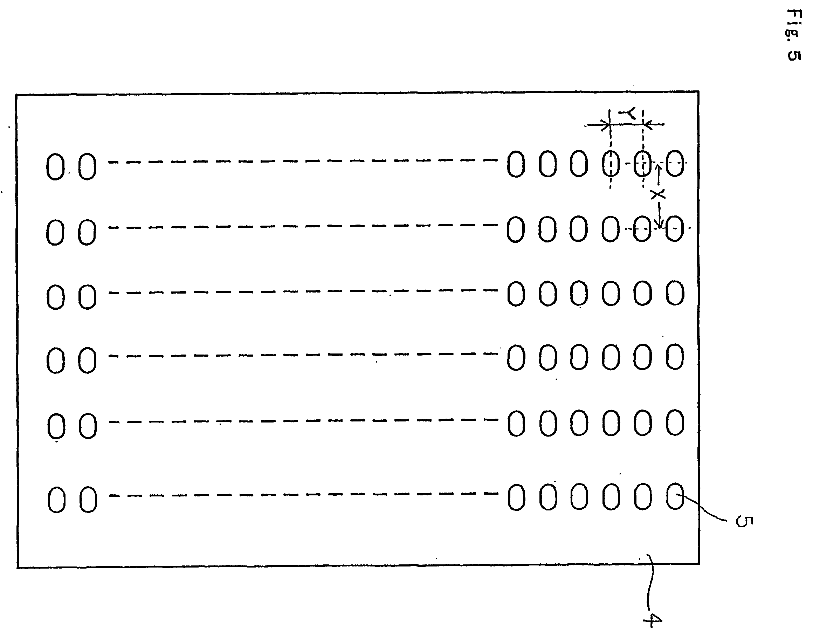

It is necessary that the water-backflow preventing layer (d) 4 is composed of a water impermeable sheet material having several columns of conduit holes 5 as shown in Fig. 5 and the total area ratio of the water penetrating holes accounts for 3% or above and below 40% of the whole area of the water-backflow preventing layer. When the ratio is below 3%, the water dropped into the water absorbent layer (a) is hard to pass through the water penetrating holes of the water-backflow preventing layer to promote unpleasant touches such as a wet touch or a cool touch of the surface, though the water backflow from the water holding layer (a) to the water absorbent layer (a) on the surface is slight. Conversely, when the total area ratio of the water penetrating holes is 40% or above, the water backflow increases in quantity and an unpleasant touch causes a problem, though transferability of water from the water absorbent layer (a) to the water holding layer (b) is good.

The size of the water penetrating holes is preferably below 5 mm, more preferably below 3 mm in the longitudinal (column) direction, as defined that the long side direction is a longitudinal direction when it is used for a diaper. The size in the (transverse) direction perpendicular to the columns is preferably 3 mm or above, more preferably 4 mm or above. When the size in the longitudinal direction of the water penetrating holes becomes 5 mm or above, water collected in the water holding layer (b) in the lower layer sometimes passes through the water penetrating holes by slight pressurization and flows back to the water absorbent layer (a) to cause unpleasant touches such as a wet touch or a cool touch. On the other hand, when the size in the transverse direction of the water penetrating holes becomes below 3 mm, rapid transfer of water from the water absorbent layer (a) to the water holding layer (b) may be difficult. As a result, a large amount of water stays in the water absorbent layer (a) on the surface and once again causes unpleasant touches such as a wet touch or a cool touch.

The column interval X and row interval Y of the water penetrating holes shown in Fig. 5 will be detailed hereinafter. Although the water transfer rate from the water absorbent layer (a) to the water holding layer (b) is improved with narrower column interval X of the water penetrating holes, the backflow of water from the water holding layer (b) is increased vice versa. The column interval X, therefore, is preferably 10 mm or above and 30 mm or below. When the column interval X exceeds 30 mm, the water absorption rate of the water absorbent layer (a) is sometimes lowered though the backflow preventing properties of water are improved.

Although the water transfer rate is improved with narrower row interval Y of the water penetrating holes shown in Fig. 5, Y is preferably 3 mm or above and 10 mm or below because the backflow preventing properties of water are lowered.

Further, although the size of the water penetrating holes is preferably below 5 mm in the longitudinal direction and is preferably 4 mm or above in the transverse direction as mentioned above, the shape of the water penetrating holes may be an optional form including a square, a circle or a slit. An ellipse, however, is most preferred by taking the durability of the shape of the water penetrating holes for a long period into consideration. This is because it is hard to cause stress concentration of external force and cracks due to the absence of corner parts as compared with the case of the water penetrating holes in a square form.

At least one layer of the water absorbent layer (a) and the water holding layer (b) is preferably bound through the water-backflow preventing layer (d) in order to improve the transferability of water from the water absorbent layer (a) to the water holding layer (b). At least one layer of the water absorbent layer (a) and the water holding layer (b) may be more preferably bound through the water penetrating holes of the water-backflow preventing layer (d).

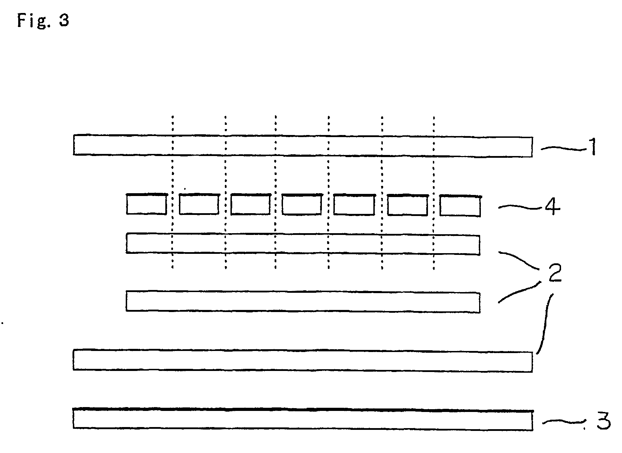

As shown in Figs. 2 and 3, the binding plays an important role in rapidly passing water dropped into the water absorbent layer (a) through the water penetrating holes of the water-backflow preventing layer (d) and in transferring the water to the water holding layer (b). The water absorbent layer (a) makes recessed parts at the binding points and initially the dropped water is concentrated there, and they assist the water in readily passing through the water penetrating holes of the water-backflow preventing layer (d). Further, the quick transference of the water is assisted by bringing the lower layer of the water absorbent layer (a) into close contact with the surface of the water holding layer (b).

The most usual method, as a specific method mentioned above for binding, is to bind with a sewing thread using a sewing machine. The method for binding, however, may be to make the water absorbent layer (a) closely adhere and join to the water holding layer (b), is not always limited to binding with a sewing thread and may be any other optional bindings.

When the water holding layer (b) bound to the water absorbent layer (a) is composed of plural layer structures, at least one layer thereof may be bound, and some layers in the plural layer structures or all the water holding layers may be bound.

Although a linear shape is simplest in the shape of binding with the sewing thread, any shape such as a curved line, a lattice and a hexagonal form may be used.

The water impermeable layer (c) and the water-backflow preventing layer (d) used in the present invention may be any combination insofar as the layers are composed of the following water impermeable sheet materials.

Namely, the layers (c) and (d) may be a film hardly permeating water such as a polyurethane film, a polyethylene film or a fluorine-based film bonded to a woven or a knitted fabric by a laminating method or the like or may be the woven or knitted fabric coated with a resin such as the urethane. The water backflow preventing layer (d), however, may be a strongly water repellent fabric having durability in washing in addition to the laminated fabric or the coated fabric, or any material containing air layers such as a double raschel knitted fabric insofar as the material is a sheetlike material which does not readily transfer water in the water absorbent layer (a) to the water holding layer (c).

Actions of the respective constituent layers on comfortable functions of the composite water absorbent sheet of the present invention will be explained hereinafter.

The water absorbent layer (a) constituting the water absorbent composite sheet has a part in quickly absorbing water, rapidly transferring the water through the water penetrating holes of the water-backflow preventing layer (d) to the water holding layer (d) and thereby always retaining the surface layer in dry touch without holding excessive water inhibiting the comfortableness.

Moreover, as described above, the role of the water-backflow preventing layer (d) consists in assisting the excessive water in the water absorbent layer (a) quickly transferring to the water holding layer (b) and preventing the water stored in the water holding layer (b) from flowing back to the surface of the water absorbent layer (a).

On the other hand, the binding of the water absorbent layer (a) through the layer (d) to the water holding layer (b), preferably the binding of the water absorbent layer (a) through the water penetrating holes of the layer (d) to the water holding layer (b), plays a role in assisting the excessive water in the water absorbent layer (a) in quickly transferring to the water holding layer (b).

In addition, the water impermeable layer (c) acts so as not to leak the water contained in the water holding layer (b) to the outside of the water absorbent composite sheet.

### Examples

The present invention will be specifically detailed by citing Examples. The composite water absorbent sheet was evaluated according to the following methods:

### (1) Water-rewet preventing property

Onto a water absorbent sheet (length 50 cm × width 25 cm), was dropped 150 cc of water from the side of the water absorbent layer (a) at a rate of 10 cc/sec. The water absorbent sheet was then allowed to stand for 30 seconds, and the part where the water was dropped, as the center, was pressurized at 98 Pa (100 g/cm²). The surface was further brought into contact with a moisture detecting paper manufactured by Toyo Roshi Kaisha Ltd. at a pressure of 49 Pa (50 g/cm²) to observe the backflow state of the water, and the palm of a hand was actually brought into contact to carry out organoleptic judgment of a wet touch due to backflow and sticking of the water on the following criteria:
ⓞ: an extremely comfortable state in a dry touch with almost no water-rewet
○ : a nearly satisfactory comfortable state with slight water-rewet
Δ : a tolerable state with a slight unpleasant touch though with somewhat water-rewet and
× : an uncomfortable state with water-rewet in an increased quantity and strong wet touch and cool touch.

### (2) Water absorbability

Onto a water absorbent sheet (length 50 cm × width 25 cm), was dropped 150 cc of water from the side of the water absorbent layer (a) at a rate of 10 cc/sec. A value obtained by measuring the time (seconds) required for special reflection (specular reflection) due to water on the surface of the water absorbent layer from the completion of the dropping to the disappearance of the reflection was called the water absorbability. The water absorbability is better with a shorter value.

### Example 1

A tricot 4-bar knitting machine was used, and a 111.1 dtex/24 filament polyester (PET, hereinafter PET means polyethylene terephthalate) yarn was used in a front bar (F). A 166.7 dtex/48 filament PET textured yarn was used in one of middle bars (M) and a 55.5 dtex/24 filament PET yarn was used in the other. A 166.7 dtex/48 filament PET textured yarn was used in a back bar (B). Thereby, the water absorbent layer (a) of the water absorbent composite sheet of the present invention was knitted.

The surface of the water absorbent layer was subjected to raising so as to provide the total number of uneven parts of 11 to 12 based on 10 cm.

(d) The water-backflow preventing layer was obtained by boring water penetrating holes, as follows, in a sheet prepared by subjecting a 32G smooth knitted fabric using an 83.3 dtex/36 filament PET yarn to laminating processing with difluoroethylene. The size of the water penetrating holes was an elliptical shape having a length (minor axis) of 3 mm and a width (major axis) of 5 mm in the case where the direction of long sides was the length when the water-backflow preventing layer was used in a diaper. The interval in the longitudinal direction of the water penetrating holes, i.e. the column interval X was 20 mm and the interval in the transverse direction, i.e. the row interval Y was 5 mm. As a result, the open area ratio was 8.0%.

(b) The water holding layer was prepared by knitting a 20G ripple knitted fabric using 95% of a No. 40 count cotton single yarn and 5% of a highly shrinkable polyester yarn having a boil-off shrinkage percentage of 30% or above.

(c) The water impermeable layer was obtained by using a sheet prepared by laminating difluoroethylene onto a smooth fabric using an 83.3 dtex/36 filament polyester yarn in the same manner as in the water-backflow preventing layer (d).

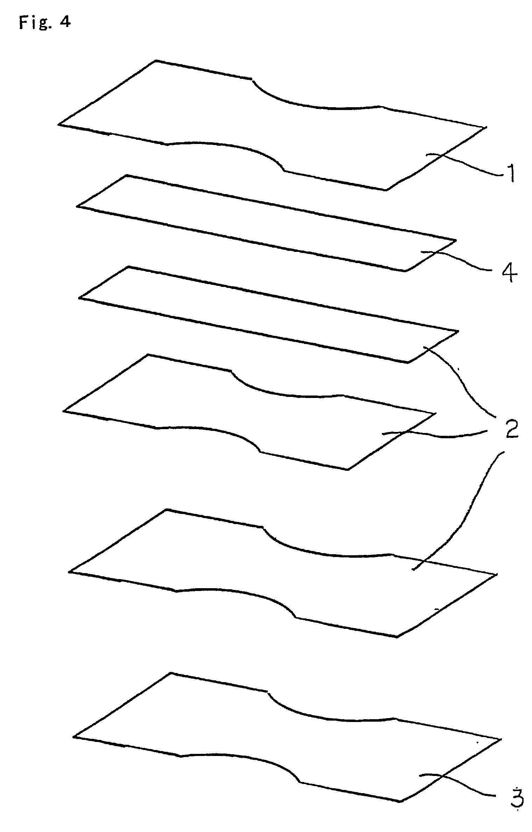

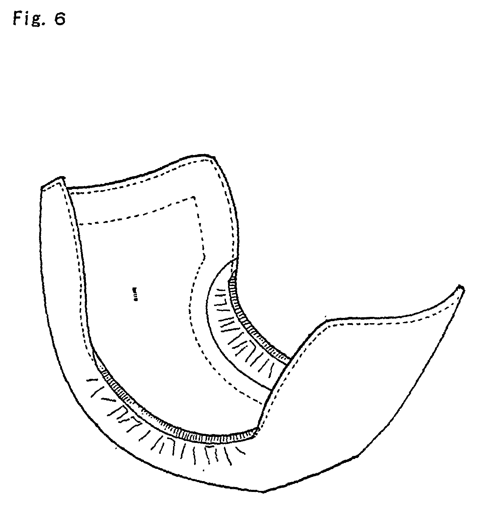

The above water absorbent layer (a), the water-backflow preventing layer (d), the water holding layer (b) and the water impermeable layer (c) were then sewed into the shape of a diaper as shown in Figs. 4 and 6.

Specifically, the periphery was sewed by a 2-needle overlock sewing machine, and stretchable peripheral tapes kept still in an extended state were then sewed onto both sides of the crotch part of the diaper by a 2-needle flat seamer sewing machine. The tension was released to thereby form a three-dimensional shape shown in Fig. 6 so that the whole diaper was brought into close contact with the crotch part of a human body.

### Example 2

The water absorbent layer (a), the water holding layer (b), the water impermeable layer (c) and the water-backflow preventing layer (d) were used in the same manner as in Example 1, and the same procedures were carried out as in Example 1, except that a three ply No. 50 count polyester filament machine sewing thread was used in one layer of the water absorbent layer (a) and the water holding layer (c) and 5 columns were randomly sewed in the longitudinal direction of the diaper at a needle stitch number of 15 needles/3 cm. The seams were not passed through the water penetrating holes in the process.

### Example 3

The water absorbent layer (a), the water holding layer (b), the water impermeable layer (c) and the water-backflow preventing layer (d) were used in the same manner as in Example 1, and the same procedures were carried out as in Example 1, except that the No. 50 count three ply polyester filament machine sewing thread was used in one layer of the water absorbent layer (a) and the water holding layer (b) and 5 columns were sewed in the longitudinal direction of the diaper at a needle stitch number of 15 needles/3 cm so that seams were passed through the water penetrating holes.

In the process, at least one layer of the water absorbent layer (a) and the water holding layer (b) was bound through the water penetrating holes of the water-backflow preventing layer (d).

### Example 4

The same procedures were carried out as in Example 3, except that only the size of the water penetrating holes of the water-backflow preventing layer (d) was changed into a circular form having a diameter of 2 mm in Example 3.

### Comparative Example 1

The same procedures were carried out as in Example 3, except that only the size of the water penetrating holes of the water-backflow preventing layer (d) was changed into a circular form having a diameter of 1 mm in Example 3. The total area ratio of the water penetrating holes herein was 0.9%.

The binding of the water absorbent layer (a) to the water holding layer (b) was performed in the same manner as in Example 3 and, however, the seams were not passed through the water penetrating holes because the seam pitch was 2 mm.

### Comparative Example 2

The same procedures were carried out as in Example 1, except that the water penetrating holes of the water-backflow preventing layer (d) were changed into a slit form having a width of 5 mm in Example 1.

Table 1 shows results of performance evaluation of composite water absorbent sheets obtained in Examples 1, 2, 3 and 4 and Comparative Examples 1 and 2.

Example 1 was in a nearly satisfactory state such that the size and column and row intervals of the water penetrating holes, i.e. the open area ratio was 8.0%, within the preferred range of the present invention, and the water absorbability was as good as 19 seconds, with slight water-rewet.

Example 2 was in a nearly satisfactory state such that the water absorbability was as quick as 15 seconds, with slight water-rewet, because the water absorbent layer (a) was bound to the water holding layer (b).

Example 3 was in an extremely comfortable state in dry touch such that the water absorbability was as quicker as 7 seconds, with hardly any water-rewet, because the water absorbent layer (b) was bound through the water penetrating holes to the water holding layer (b).

Example 4 was in a nearly satisfactory state such that the water absorbability was as good as 12 seconds, with slight water-rewet, though the water absorbability was somewhat slower than that of Example 3 because the size of the water penetrating holes was decreased to a circular form having a diameter of 2 mm.

Comparative Example 1, however, was in a state such that the water absorbability was as slow as 35 seconds, the water transferability from the water absorbent layer (a) to the water holding layer (b) was bad and the water-rewet preventing property was at a level providing an unpleasant touch, because the size of the water penetrating holes was 1 mm and the open area ratio was as low as 0.9%.

Although the same layer construction as in Example 1 was used, Comparative Example 2 was in a state such that the water-rewet preventing property was deteriorated, though the rate at which the water in the water absorbent layer transferred to the water holding layer was 14 seconds within the tolerance, according to that the open area ratio was increased to 40.0% because the water penetrating holes were changed into slits having a width of 5 mm. In addition, water readily flowed back from wide openings when a pressure was applied and resulted in promotion of an unpleasant touch. As a result, the comfortableness to wear of Comparative Example 2 was deteriorated to an unpleasant level incomparable to Examples 1 to 4 of the present invention.

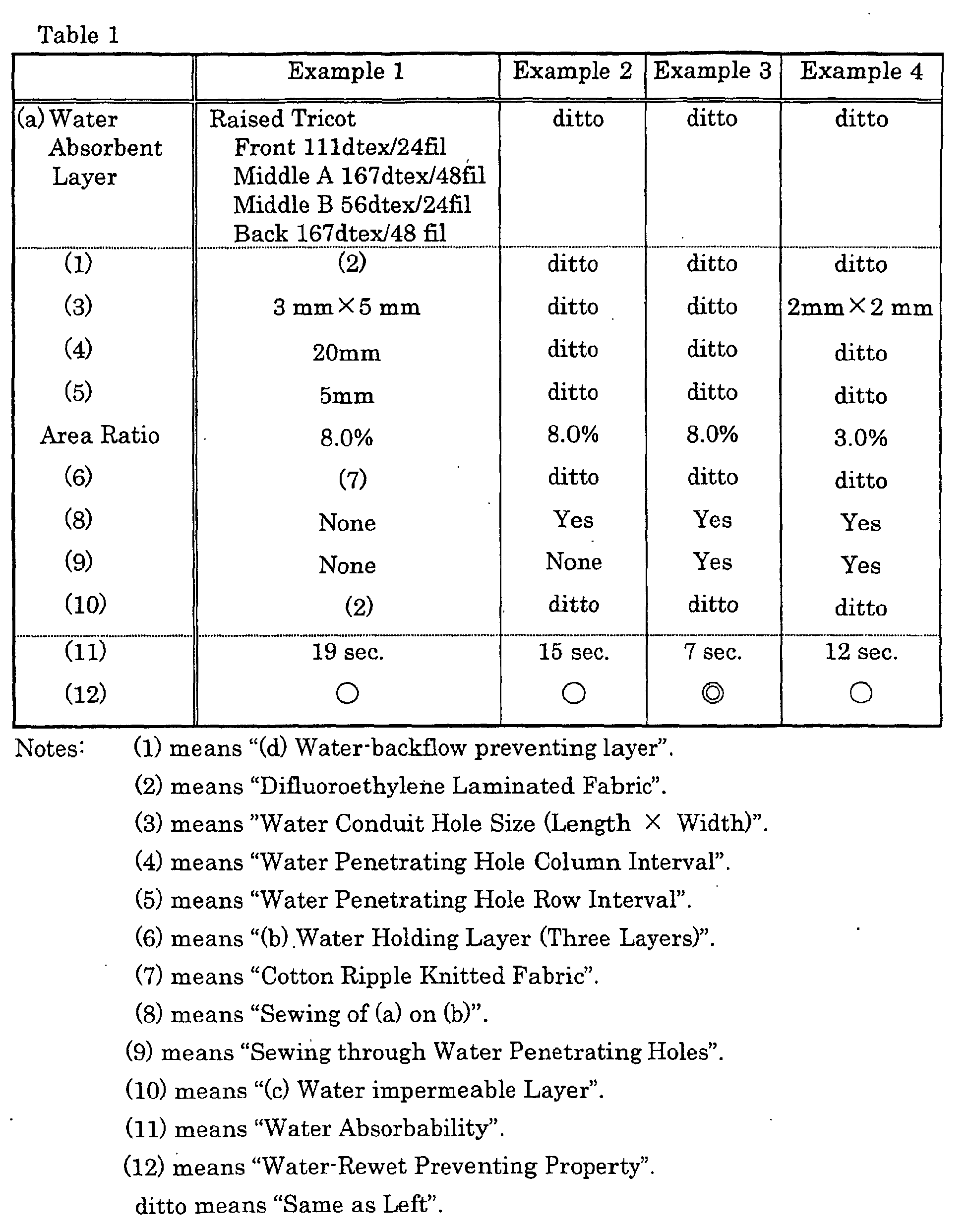

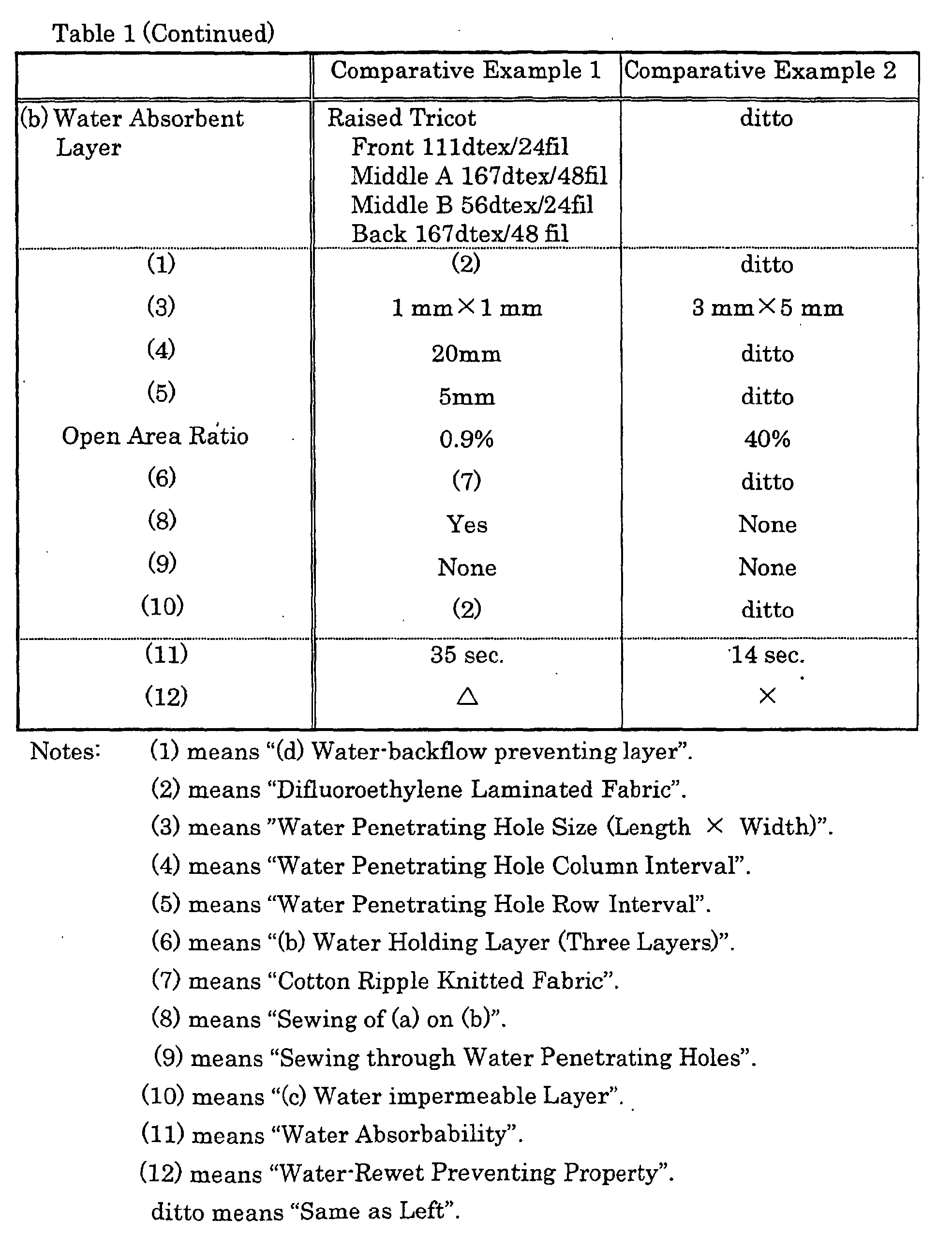

### Industrial Applicability

As described in the present invention, there can be obtained a comfortable composite water absorbent sheet which is a water absorbent sheet comprising a water absorbent layer, a water holding layer and a water impermeable layer successively arranged from the skin side, wherein the composite water absorbent sheet has a sufficient amount of water absorption with slight water-rewet to the skin by arranging a water-backflow preventing layer comprising a water impermeable sheet material having water penetrating holes between the water absorbent layer and the water holding layer and providing the total area ratio of the water penetrating holes accounting for 3% or above and below 40% of the whole area of the water-backflow preventing layer. The water absorbent composite sheet is washable, repetitively usable and suitably usable as diapers for nursing care of infants and the aged, urine pads, sheets and further urine absorbent sheets for pets.

## Claims

1. A water absorbent composite sheet which is a water absorbent sheet comprising (a) a water absorbent layer, (b) a water holding layer and (c) a water impermeable layer successively arranged from the skin side and **characterized in that** (d) a water-backflow preventing layer comprising a water impermeable sheet having water penetrating holes is arranged between the layer (a) and the layer (b) and the total area ratio of the water penetrating holes accounts for 3% or above and below 40% of the whole area of the layer (d).

2. The water absorbent composite sheet according to claim 1, wherein at least one layer of the layer (a) and the layer (b) is bound through the layer (d).

3. The water absorbent composite sheet according to claim 2, wherein at least one layer of the layer (a) and the layer (b) is bound through the water penetrating holes of the layer (d).

4. The water absorbent sheet according to claim 2 or 3, wherein the binding of the layer (a) and the layer (b) is a binding with a sewing thread.
